# EUROPÄISCHE PATENTSCHRIFT

(11) **EP 1 453 473 B1**
(45) Veröffentlichungstag und Bekanntmachung des Hinweises auf die Patenterteilung: **03.03.2010**
(21) Anmeldenummer: 02799052.2
(22) Anmeldetag: 04.12.2002
(51) Int. Cl.: A61K 8/37, A61Q 5/00, A61Q 19/00

(54) **EMOLLIENTS UND KOSMETISCHE ZUSAMMENSETZUNGEN ENTHALTEND 2-METHYL-1,3-PROPANDIOLDIESTER**
EMOLLIENTS AND COSMETIC COMPOSITIONS COMPRISING 2-METHYL-1,3-PROPANEDIESTERS
EMOLLIENTS ET COMPOSITIONS COSMETIQUES COMPRENANT UN DIESTER DE 2-METHYL-1,3-PROPANEDIOL

(30) Priorität: 11.12.2001 DE 10160682
(43) Veröffentlichungstag der Anmeldung: 08.09.2004
(73) Patentinhaber: Cognis IP Management GmbH, 40589 Düsseldorf (DE)
(72) Erfinder: PRINZ, Daniela, 41542 Dormagen (DE); WESTFECHTEL, Alfred, 40724 Hilden (DE)
(86) Internationale Anmeldenummer: PCT/EP2002/013695
(87) Internationale Veröffentlichungsnummer: WO 2003/053373

(56) Entgegenhaltungen:
- EP-A- 0 103 893
- DE-A- 19 705 573
- FR-A- 1 526 996
- US-A- 5 080 901
- DATABASE CAPLUS [Online] CHEMICAL ABSTRACTS SERVICE, COLUMBUS, OHIO, US; 8. März 1982 (1982-03-08), THOMAS, ALAN F.: "The occurrence of some novel diesters in Roman camomile oil" XP002234228 accession no. STN Database accession no. 1982:74484 & HELVETICA CHIMICA ACTA, Bd. 64, Nr. 7, 1981, Seiten 2397-2400, ISSN: 0018-019X
- PATENT ABSTRACTS OF JAPAN Bd. 008, Nr. 150 (C-233), 12. Juli 1984 (1984-07-12) & JP 59 055853 A (DAICEL KK;OTHERS: 01), 31. März 1984 (1984-03-31) in der Anmeldung erwähnt

## Beschreibung

### Gebiet der Erfindung

Die Erfindung betrifft neuartige Ölkörper auf Esterbasis, die leicht in kosmetische und pharmazeutische Zubereitungen einarbeitbar sind, eine gute dermatologische Verträglichkeit aufweisen und kosmetischen Formulierungen ein besonders leichtes Hautgefühle vermitteln.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologische Verträglichkeit zu erfüllen, werden kontinuierlich neue Ölkörper und Emulgator-Gemische entwickelt und getestet.

Aufgabe der vorliegenden Erfindung war es, neue Emollients für kosmetische Applikationen zur Verfügung zu stellen, die ähnlich Esterölen eine gewisse Polarität mit vorwiegend lipohilen Eigenschaften aufweisen und in eine Vielzahl kosmetischer Formulierungen einarbeitbar sind. Überraschenderweise wurde gefunden, dass Diester des 2-Methly-1,3-Propandiols dermatologisch gut verträglich sind, zu sensorisch leichten Produkten führen und auch leicht in Kaltprozessen einarbeitbar sind.

### Beschreibung der Erfindung

Gegenstand der Erfindung ist die Verwendung von 2-Methyl-1,3-Propandioldiestern der Formel (I) 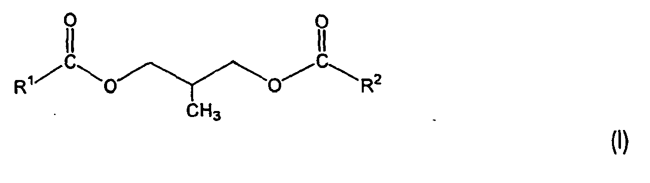 in der R¹ und R² unabhängig voneinander für eine gesättigte oder ungesättigte, verzweigte oder unverzweigte C₆-C₃₀-Alkylgruppe oder einen aromatischen Rest stehen. Besonders bevorzugt ist die Verwendung solcher Diester, in denen R¹ und R² unabhängig voneinander für eine lineare, gesättigte C₁₂-C₂₄-Alkylgruppe stehen. Als aromatische Reste sind beispielsweise Benzoesäurereste geeignet. Vorzugsweise sind R¹ und R² identisch, so dass es sich um symmetrische Diester handelt.

Diester des 2-Methyl-1,3-Propandiols sind aus der JP-A 59055853 als Schmiermittel und Lösungsmittel für Farben bekannt.

Überraschenderweise zeigte sich, dass die Verbindungsklasse eine Kombination aus polaren und lipophilen Eigenschaften aufweist, die sie besonders geeignet als Emollient für kosmetische Applikationen macht.

Die Verbindungen können nach gängigen Veresterungsverfahren des Standes der Technik hergestellt werden, d. h. beispielsweise ohne Katalysator oder unter Säurekatalyse. Die Ausbeuten sind jedoch in diesem Fall nicht zufriedenstellend und oft entstehen unerwünschte Nebenprodukte, deren Abtrennung Schwierigkeiten bereitet. Geeigneter ist ein Veresterungsverfahren, bei dem Zinnkatalysatoren eingesetzt werden, in einem Verfahren zur Herstellung von 2-Methyl-1,3-Propandioldiestem gemäß Formel (I), in der R¹ und R² unabhängig voneinander für eine gesättigte oder ungesättigte, verzweigte oder unverzweigte C₁₂-C₂₄-Alkylgruppe oder einen aromatischen Rest stehen, wobei man:
(a) 2-Methyl-1,3-Propandiol in Gegenwart eines Zinn-Katalysators mit einer Carbonsäure R¹-COOH und/oder R²-COOH im Überschuss solange verestert, bis die Wasserbildung beendet ist
(b) das Reaktionsprodukt über Tonsil abfiltriert und
(c) den Diester durch fraktionierte Destillation reinigt.

Als Zinn-Katalysatoren geeignet sind beispielsweise Zinnoxalat (z. B. Fascat^{®} 2001), Zinnoxid (SnO, Fascat^{®} 2000) sowie Zinn-IV-Katalysatoren wie Dibutylzinndiacetat Fascat^{®} 4200), Dibutlyzinnoxid (Fascat^{®} 4201), und Dibutylzinnlaurat (Fascat^{®} 4202). Erfindungsgemäß bevorzugt geeignet ist Zinnoxid (SnO).

### Kosmetische/pharmazeutische Zubereitungen

Die erfindungsgemäßen Emollients erlauben die Herstellung stabiler kosmetischer und pharmazeutischer Emulsionen. Ein weiterer Gegenstand der vorliegenden Erfindung sind daher kosmetische und/oder pharmazeutische Zubereitungen, die wenigstens einen 2-Methyl-1,3-Propandioldiester enthalten gemäß Anspruch 4, vorzugsweise einen 2-Methyl-1,3-Propandiol-C₁₂-C₂₄-diester, bei dem R¹ und R² identisch sind. Vorzugsweise ist der 2-Methyl-1,3-Propandioldiester in einer Menge von 0,1 - 50 Gew.-% und insbesondere 0,1 bis 40 Gew.-% in den Zubereitungen enthalten. Besonders bevorzugt sind Einsatzmengen von 1- 30 Gew.-% und insbesondere 1 - 20 Gew.-%.

Vorzugsweise handelt es sich hierbei um Formulierungen zur Körperpflege, z. B. Cremes, Milchen, Lotionen, sprühbare Emulsionen, Produkte zur Eliminierung des Körpergeruchs etc. Die erfindungsgemäßen Verbindungen lassen sich auch in tensidhaltigen Formulierungen wie z. B. Schaum- und Duschbädem, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe etc., die nachstehend exemplarisch aufgelistet sind.

### Tenside

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 0,1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für **amphotere bzw. zwitterionische Tenside** sind Alkylbetaine, Alkylamidobetaine, Aminopropionate, Aminoglycinate, lmidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe weiterer Ölkörper und Emollients, die dazu beitragen die sensorischen Eigenschaften weiter zu optimieren. Die Ölkörper sind üblicherweise in einer Gesamtmenge von 0,1 - 50 Gew.-%, vorzugsweise 5 - 25 Gew.-% und insbesondere 5 - 15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen, Siliconöle (Cyclomethicone, Siliciummethicontypen u. a.) und/oder aliphatische bzw. naphthenische Kohlenwasserstoffe, wie z. B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest
➢ Alkyloligoglykoside mit 8 bis 22 Kohlenstoffatomen im Alkylrest und deren ethoxylierte Analoga
➢ Anlagerungsprodukte von 1 bis 15 Mol Ethylenoxid an Ricinusöl und/oder gehärtetes Ricinusöl
➢ Anlagerungsprodukte von 15 bis 60 Mol Ethylenoxid an Ricinusöl und/oder gehärtetes Ricinusöl
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
➢ Rartialester-von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z. B. Sorbit), Alkylglucosiden (z. B. Methylglucosid, Butylglucosid, Laurylglucosid) sowie Polyglucosiden (z. B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze
➢ Wollwachsalkohole
➢ Polysiloxan-Polyalkyl-Polyether-Copolymere bzw. entsprechende Derivate
➢ Block-Copolymere z. B. Polyethylenglycol-30 Dipolyhydroxystearate
➢ Polymeremulgatoren, z. B. Pemulen-Typen (TR-1 ,TR-2) von Goodrich
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyvricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphothere und kationische Emulgatoren

Weiterhin konnen als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

Bevorzugt im Sinne der Erfindung sind kosmetische und/oder pharmazeutische Zubereitungen, die
(a) 0,1 - 50 Gew.-% wenigstens eines 2-Methyl-1,3-Propandioldiesters gemäß Anspruch 1
(b) 0,1 - 20 Gew.-% Tenside und/oder Emulgatoren und/oder Coemulgatoren
(c) 0,1 - 40 Gew.-% weiterer Ölkörper und
(d) 0 - 98 Gew.-% Wasser
bezogen auf die Gesamtzusammensetzung enthalten.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmonoldilaurat, -palmitat oder -stearat kommen hierfür in Frage. Als Wachse kommen u. a. natürlichen Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z. B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon.

Als besonders wirkungsvoll haben sich auch Bentonite, wie z. B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### Polymere

Geeignete kationische Polymere sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z. B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z. B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, kationischer Guar-Gum, wie z. B. Jaguar® CBS, Jaguar® C-17, Jaguar® C-16 der Firma Celanese, quatemierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.-Butylaminoethylmethacrylat/2-Hydroxypropvlmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
➢ 3-Benzylidencampher bzw. 3-Benzylidennorcampher und dessen Derivate, z. B. 3-(4-Methylbenzyliden)campher wie in der EP 0693471 B1 beschrieben
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene)
➢ Ester der Salicylsäure, vorzugsweise Salicylsäure-2-ethylhexylester, Salicylsäure-4-iso-propylbenzylester, Salicylsäurehomomenthylester
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
➢ Ester der Benzalmalonsäure, vorzugsweise 4-Methoxybenzmalonsäuredi-2-ethylhexyl-ester
➢ Triazinderivate, wie z. B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB)
➢ Propan-1,3-dione, wie z. B. 1-(4-tert.Butylphenyl)-3-(4'methoxyphenyl)propan-1,3-dion
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze
➢ Sulfonsäurederivate von Benzophenonen, vorzugsweise 2-Hydroxy-4-methoxybenzo-phenon-5-sulfonsäure und ihre Salze
➢ Sulfonsäurederivate des 3-Benzylidencamphers, wie z. B. 4-(2-Oxo-3-bomylidenmethyl) benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Besonders günstige Kombinationen bestehen aus den Derivate des Benzoylmethans,, z. B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d. h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Camosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl- γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z. B. ZnO, ZnSO₄) Selen und dessen Derivate (z. B. Selen-Methionin), Stilbene und deren Derivate (z. B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)hamstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe
➢ Ölkomponenten
➢ nichtionische Emulgatoren
➢ Coemulgatoren
➢ Konsistenzgeber
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z. B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z. B. Konservierungsmittel, wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionic acid-ethyl ester), welches unter der Bezeichnung Insect Repellent^{®} 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Typische Beispiele sind:

➢ Glycerin
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton
➢ technische Oligoglyceringemische mit einem Eigenkondensationsgrad von 1,5 bis 10 wie etwa technische Diglyceringemische mit einem Diglyceringehalt von 40 bis 50 Gew.-%
➢ Methyolverbindungen, wie insbesondere Trimethylolethan, Trimethylolpropan, Trimethylolbutan, Pentaerythrit und Dipentaerythrit
➢ Niedrigalkylglucoside, insbesondere solche mit 1 bis 8 Kohlenstoffen im Alkylrest, wie beispielsweise Methyl- und Butylglucosid
➢ Zuckeralkohole mit 5 bis 12 Kohlenstoffatomen, wie beispielsweise Sorbit oder Mannit
➢ Zucker mit 5 bis 12 Kohlenstoffatomen, wie beispielsweise Glucose oder Saccharose
➢ Aminozucker, wie beispielsweise Glucamin
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015 Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Erfindungsgemäßes Beispiel: 2-Methyl-1,3-Propandioldilaurylester:

### Umsetzung:

| | | |
|---|---|---|
| I | 1 mol = 90,1 g | 2-Methyl-1,3-Propandiol |
| II | 2,5 mol = 500,8 g | Laurinsäure |
| III | 5,9 g | SnO |

In einem 1-L-Kolben mit Wasserabscheider und N₂-Begasung lässt man die Mischung (I + II + III) bei 220 °C mehrere Stunden reagieren, bis kein Wasser mehr entsteht. Nach Abkühlung des Reaktionsgemisches, wird Tonsil zugegeben, und der Katalysator und das Tonsil anschließend durch Filtration über eine D2-Glasfilternutsche entfernt. Das Rohprodukt aus 1,2 % Monoester, 87,6 % Diester und 9,6 % Laurinsäure wird bei 230°C Sumpf- und 212°C Brüdentemperatur bei 0,03 mPa über eine 250-mm-Vigreux-Kolonne destilliert, um den Anteil des Diesters auf 98,0 % zu erhöhen.

## Patentansprüche

1. Verwendung von 2-Methyl-1,3-Propandioldiestem der Formel (I) 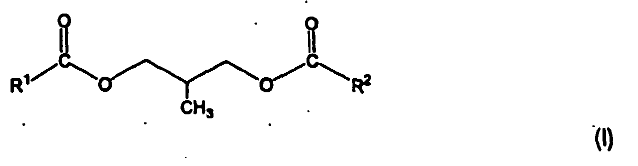 in der R¹ und R² unabhängig voneinander für eine gesättigte oder ungesättigte, verzweigte oder unverzweigte C₆-C₃₀ Alkylgruppe oder einen aromatischen Rest stehen, in kosmetischen und/oder pharmazeutischen Zubereitungen.

2. Verwendung gemäß Anspruch 1, **dadurch gekennzeichnet, dass** R¹ und R² unabhängig voneinander für eine lineare, gesättigte C₁₂-C₂₄-Alkylgruppe stehen.

3. Verwendung gemäß Anspruch 1 und/oder 2 als Emollient.

4. Kosmetische und/oder pharmazeutische Zubereitung enthaltend
(e) 0,1 - 50 Gew.-% wenigstens eines 2-Methyl-1,3-Propandioldiesters gemäß Anspruch 1
(f) 0,1 - 20 Gew.-% Tenside und/oder Emulgatoren und/oder Coemulgatore
(g) 0,1 - 40 Gew.-% weiterer Ölkörper
(h) 0 - 98 Gew.-% Wasser.
bezogen auf die gesamtzusammensetzung

## Claims

1. The use of 2-methylpropane-1,3-diol diesters corresponding to formula (I): 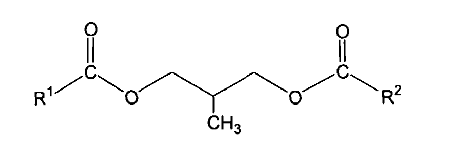 in which R¹ and R² independently of one another represent a saturated or unsaturated, branched or unbranched C₆-₃₀ alkyl group or an aromatic residue,
in cosmetic and/or pharmaceutical preparations.

2. The use claimed in claim 1, **characterized in that** R¹ and R² independently of one another represent a linear, saturated C₁₂₋₂₄ alkyl group.

3. Use according to claim 1 and/or 2 as emollient.

4. A cosmetic and/or pharmaceutical preparation containing
(a) 0.1 to 50% by weight of at least one 2-methylpropane-1,3-diol diester according to claim 1,
(b) 0.1 to 20% by weight of surfactants and/or emulsifiers and/or co-emulsifiers,
(c) 0.1 to 40% by weight of other oil components and
(d) 0 to 98% by weight of water.
based on the cosmetic as a whole.

## Revendications

1. Utilisation de diesters de 2-méthyl-1,3-propanediol de formule (I) 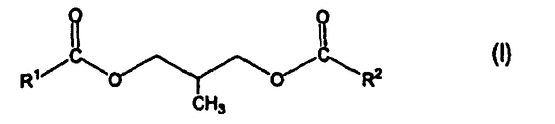 dans laquelle R¹ et R² représentent, indépendamment l'un de l'autre, un groupe C₆-C₃₀-alkyle saturé ou insaturé, ramifié ou non ramifié ou un radical aromatique, dans des préparations cosmétiques et/ou pharmaceutiques.

2. Utilisation selon la revendication 1, **caractérisée en ce que** R¹ et R² représentent, indépendamment l'un de l'autre, un groupe C₁₂-C₂₄-alkyle linéaire, saturé.

3. Utilisation selon la revendication 1 et/ou 2 comme émollient.

4. Préparation cosmétique et/ou pharmaceutique, contenant
(e) 0,1-50% en poids d'au moins un diester de 2-méthyl-1,3-propanediol selon la revendication 1
(f) 0,1-20% en poids d'agents tensioactifs et/ou d'émulsifiants et/ou de co-émulsifiants
(g) 0,1-40% en poids d'autres corps huileux
(h) 0-98% en poids d'eau,
par rapport à la composition totale.
